# NEUE EUROPÄISCHE PATENTSCHRIFT

(11) **EP 2 226 058 B2**
(45) Veröffentlichungstag und Bekanntmachung des Hinweises auf die Entscheidung über den Einspruch: **20.01.2016**
(45) Hinweis auf die Patenterteilung: 18.01.2012
(21) Anmeldenummer: 10004861.0
(22) Anmeldetag: 28.03.1995
(51) Int. Cl.: A61J 1/00, B32B 7/06, B65D 81/32

(54) **Polymerer medizinischer Mehrkammerbeutel mit durch abziehbarer Dichtung getrennten Kammern**
Medical multiple chamber polymer bag having compartments separated by a pealable seal
Sachet polymère à chambres multiples d'usage médical, avec compartiments séparés par une fermeture étanche thermoscellée pelable

(30) Priorität: 29.03.1994 DE 4410876
(43) Veröffentlichungstag der Anmeldung: 08.09.2010
(62) Teilanmeldung aus: 04003975.2
(73) Patentinhaber: Fresenius AG, 61352 Bad Homburg v.d.H. (DE)
(72) Erfinder: Kreischer, Thomas, 66113 Saarbrücken (DE); Heilmann, Klaus, 66606 St. Wendel (DE); Zimmermann, Michael, 66606 St. Wendel (DE); Nicola, Thomas, 57350 Spicheren (FR)
(74) Vertreter: Ricker, Mathias

(56) Entgegenhaltungen:
- EP-A- 0 345 774
- EP-A- 0 513 364
- WO-A1-92/02271
- WO-A1-93/21890
- DE-A1- 3 238 649
- DE-A1- 3 700 713
- JP-Y2- S6 231 401
- US-A- 4 519 499
- US-A- 5 176 634

## Beschreibung

Die Erfindung betrifft einen medizinischen Mehrkammerbeutel zur Herstellung von medizinischen Mischlösungen, der mindestens 3 Kammern aufweist, die durch einen verschweißten, zu öffnenden Trennbereich voneinander getrennt sind und im Außenrandbereich verschweißt sind, wobei in der Schweißnaht des Außenrandbereiches mindestens ein Stutzen in mindestens einer Kammer vorgesehen ist..

Medizinische Mehrkammerbeutel werden seit Jahren zur Herstellung von Mischlösungen eingesetzt. Die bekannten Mehrkammerbeutel weisen dabei als Trennvorrichtung zwischen den Kammern verschiedene Systeme auf.

Eines dieser Systeme verwendet Trennbrechteile aus starren, brechfähigen Materialien. Diese haben den Vorteil einer weitgehend universellen Verwendbarkeit, weisen jedoch insofern Nachteile auf, als sie nur eine begrenzte Brech- bzw. Mischöffnung freigeben und es beim Aufbrechen der Trennvorrichtung zu unerwünschter Partikelbildung kommen kann.

Ein ähnliches System, das im Kammertrennbereich eine Sollbruchstelle aufweist, die dadurch zerbrochen werden kann, daß beispielsweise auf eine Kammer Druck ausgeübt wird, ist in der EP 0 378 183 offenbart, wobei im Bereich der Sollbruchstelle zweckmäßigerweise noch ein Peel-Streifen eingeschweißt ist.

Eine zweite Art von Trennvorrichtung ist als Abquetschsystem ausgebildet, das beispielsweise mittels äußerer Klammern die Folien der Beutel zur Abdichtung abdrückt und sich vor Anwendung öffnen läßt. Die Vorteile derartiger Abquetschsysteme liegen in der möglichen großen Mischöffnung, die sich in der Regel partikelfrei erzeugen läßt. Jedoch sind derartige Systeme insofern nachteilig, als sie auf Folienmaterialien für die Beutel eingeschränkt sind, die eine hohe Elastizität und Temperaturbeständigkeit haben müssen, um die gewünschte Trennwirkung erfüllen zu können. Oft weisen solche Systeme Undichtigkeiten auf. Um diese zu vermeiden, ist es erforderlich, die Toleranzen der Beuteldicke und des Abquetschsystems genau aufeinander abzustimmen.

Um bei Abquetschsystemen nicht auf bestimmte Folienarten als Material für den Mehrkammerbeutel beschränkt zu sein und um die Trennwirkung zu verbessern, wird gemäß der DE 41 07 223 im Bereich der späteren Mischöffnung ein elastischer Folienstreifen angeordnet, der bei Betätigung der Abquetscheinrichtung als Dichtung dient. Aufgrund der Abquetschvorrichtung und des Folienstreifens weist dieses System den Nachteil auf, daß es sehr aufwendig ausgestaltet ist.

US 4519 499 offenbart einem medizinischen Dreikammerbentel mit selektiv an öffnenden Schweißnähten und 3 Ausgängen.

Es ist daher Aufgabe der Erfindung, eine weitere Folie für einen medizinischen Mehrkammerbeutel zur Verfügung zu stellen, die beim Verschweißen bei einer ersten, niederen Temperatur eine peelfähige Naht und beim Verschweißen bei einer zweiten Temperatur eine feste Naht ergibt.

Diese Aufgabe wird erfindungsgemäß u.a. dadurch gelöst, daß die den Kammern zugewandte Schicht der Mehrschichtfolie welche die Schweißnaht bildet im wesentlichen zwei Komponenten aufweist, nämlich ein Matrix-Polymer und eine Phasen-Polymer.

Der überraschende Vorteil des Mehrkammerbeutels aus der erfindungsgemäßen Folie liegt darin, daß es durch das den Kammern zugewandte polymere Material möglich ist sowohl untrennbare Schweißnähte in dem Außenrandbereich als auch nachträglich wieder trennbare Schweißnähte im Kammertrennbereich auszubilden. Eine Schweißnaht im Kammertrennbereich sollte dabei mit einer Kraft im Bereich von 5 bis 20 N trennbar sein. Ist die Schweißnaht mit einer Kraft von weniger als 5 N trennbar, ist keine sichere Trennung der Kammern möglich, da die Verbindung sich dann beispielsweise durch leichte Erschütterungen beim Transport, die Druck auf eine oder mehrere Kammern ausüben, von alleine lösen kann. Bei einer Kraft von über 20 N ist die Schweißnaht nur noch sehr schwer trennbar. Es besteht die Gefahr, daß nicht die Schweißnaht, sondern die Folie reißt und damit der Beutel undicht wird.

Darüber hinaus sollte die Trennkraft der Schweißnähte so hoch sein, daß der Beutel eine Fallfestigkeit aus zwei Meter Höhe aufweist.

Der oben beschriebene Mehrkammerbeutel aus der erfindungsgemäßen gestattet eine sehr einfache Ausbildung, da weder Trennbrechteile noch Abquetschvorrichtungen erforderlich sind. Auch werden keine zusätzlichen Dichtungs- und Folienmaterialien im Kammertrennbereich benötigt.

Trotzdem wird eine sichere Trennung der Kammern erreicht, wobei die Trennung leicht zu öffnen ist und zudem noch einen möglichst großen Querschnitt freigibt.

Zur Herstellung des Mehrkammerbeutels werden vorzugsweise sogenannte Blasoder auch Schlauchfolien verwendet. Diese können gegebenenfalls gefaltet werden und müssen lediglich an zwei Seiten des Außenrandes, nämlich den beiden Öffnungen, verschlossen, d.h. verschweißt werden. Es ist aber auch die Verwendung von einzelnen Folienblättern denkbar, die aber umlaufend verschweißt werden müssen.

Als polymeres Material für Mehrkammerbeutel wird im allgemeinen eine Mehrschichtfolie eingesetzt. In einer bevorzugten Ausführungsform ist die Mehrschichtfolie eine coextrudierte Mehrschichtfolie. Des weiteren bevorzugt ist, daß die Mehrschichtfolie zwei bis sieben Schichten, aufweist. Es ist aber auch eine Monofolie als polymeres Material für den Mehrkammerbeutel denkbar.

Die Materialien der Mehrschichtfolie werden dabei so ausgewählt, daß der Beutel möglichst durchsichtig und flexibel, insbesondere aber heiß sterilisierbar und biokompatibel ist. Aus Biokompatibilitätsgründen und Umweltgesichtspunkten ist die Verwendung von PVC, das immer einen gewissen Anteil an Weichmachern aufweist, zumindest in der Innenschicht ausgeschlossen. Aus den gleichen Gründen sollten auch Materialien, wie z.B. Haftvermittler, die in das Beutelinnere diffundieren können, ausgeschlossen werden.

Für bestimmte Anwendungen ist es darüber hinaus notwendig, daß die Mehrschichtfolie eine Gasbarriere für Sauerstoff und Kohlendioxid sowie eine Wasserdampfbarriere aufweist, die eine Diffusion dieser Gase sowohl in, als auch aus dem Beutel verhindert.

Die den Kammern zugewandte Schicht der Mehrschichtfolie, im folgenden als Innenschicht bezeichnet, weist im wesentlichen zwei Komponenten auf, nämlich ein Matrix-Polymer und ein Phasen-Polymer. Das System aus Matrix- und Phasen-Polymer wird im folgenden als Matrix-Phasen-Polymer-System bezeichnet.

Als Matrix-Polymere können Polymere mit hohem Anregungs- bzw. Schmelztemperaturbereich, wie z.B. Polyethylen-Homopolymer, Polyethylen-Copolymere, Polypropylen-Homopolymer und Polypropylen-Copolymer verwendet werden. Polyethylen wird dabei als High-Density-Polyethylen (HDPE) oder Linear-Low-Density-Polyethylen (LLDPE) eingesetzt. Von den genannten Matrix-Polymeren ist das Polypropylen-Copolymer bevorzugt. Besonders bevorzugt ist ein Polypropylen-Random-Copolymer.

Als Phasen-Polymere können Polymere mit niedrigem Anregungs- bzw. Schmelztemperaturbereich, wie z.B. Styrol-Ethylen/Butylen-Styrol-Triblock-Polymer (SEBS), Styrol-Ethylen/Butylen-Styrol-Triblock-Polymer (SEBS) mit Styrol-Ethylen/Butylen-Diblock-Anteil (SEB), Styrol-EthylentPropylen-Styrol-Triblock-Polymer (SEPS), Styrol-Butadien-Styrol-Triblock-Polymer (SBS) und/oder Styrol-Isopren-Styrol-Triblock-Polymer (SIS) sowie Low-Density-Polyethylen (LDPE), Linear-Low-Density-Polyethylen (LLDPE), Terpolymere aus Ethylen, Propylen und einem nichtkonjugierten Dien (EPDM) und/oder Ethylen-α-Olefin-Copolymer verwendet werden. Vorzugsweise wird SEBS eingesetzt. Der Anteil des Phasen-Polymers in der Innenschicht sollte dabei im Bereich von 1 bis 40 Gew.-%, bezogen auf das gesamte Matrix-Phasen-Polymer-System, liegen.

Das Phasen-Polymer kann zusätzlich noch ein Verarbeitungshilfsmittel aufweisen. Dieses Hilfsmittel wird eingesetzt, um die Viskosität des Polymeren einzustellen. Die Menge des Hilfsmittels sollte dabei im Bereich von 1 bis 15 Gew.-%, bezogen auf die Menge des Phasen-Polymers, liegen. Als Verarbeitungshilfsmittel kann beispielsweise medizinisches Weißöl verwendet werden.

Die Schweißnaht des Außenrandbereiches weist mindestens einen Stutzen in mindestens einer Kammer auf, wobei dieser Stutzen ein Auslaßstutzen ist. Es ist aber auch möglich, daß dieser Auslaßstutzen gleichzeitig ein Füllstutzen ist.

Bevorzugt ist jedoch, daß zu dem Auslaßstutzen jede zu füllende Kammer im Außenrandbereich zusätzlich noch einen Füllstutzen aufweist. Die Füllstutzen sind aber nicht zwingend notwendig, denn es ist auch denkbar, beispielsweise im Fall des Zweikammerbeutels zuerst den Kammertrennbereich zu verschweißen und dann jeweils eine Kammer zu füllen und anschließend den jeweiligen Außerrandbereich zu verschweißen.

Durch Bildung einer zusätzlichen trennbaren Schweißnaht wird der Auslaßstutzen von der Beutelfüllung abgetrennt. Dies führt zu einem Dreikammerbeutel, der zwei zu füllende Kammern mit jeweils einem Füllstutzen und eine leere Kammer mit einem Auslaßstutzen aufweist. Auf diese Weise ist es möglich, zuerst die Lösungen der beiden gefüllten Kammern zu mischen und anschließend die Schweißnaht, die den Auslaßstutzen abtrennt, zu öffnen, um dadurch den Beutelinhalt freizugeben. Der Vorteil dieses Beutels liegt darin, daß es dadurch möglich ist, das nachteilige Abbrechventil im Auslaßstutzen zu sparen.

Um zu erreichen, daß zuerst der Kammertrennbereich zwischen den beiden gefüllten Kammern geöffnet wird die Schweißnaht im Kammertrennbereich zwischen den beiden zu füllenden Kammern so ausgebildet, daß die Schweißnaht zuerst an einer bestimmten Stelle geöffnet werden kann. Dies kann dadurch erreicht werden, daß ein Teil der Schweißnaht, im Kammertrennbereich zwischen den beiden zu füllenden Kammern beispielsweise in V-Form ausgebildet ist. In diesem Fall öffnet sich die Schweißnaht zuerst an der Spitze des V's, da hier die aufzuwendende Kraft am geringsten ist. Die äußere Wandung wird deshalb vorzugsweise gerade in diesem Bereich mit einer Aufreißlasche, vorzugsweise mit zwei Aufreißlaschen, versehen.

Mit Hilfe dieser AufreiBlasche(n) ist es dann sehr einfach möglich, nach einer erfolgten Durchmischung des Inhalts der beiden gefüllten Kammern, auch den Teil der Schweißnaht des Kammertrennbereichs zu öffnen, der die leere Kammer, die den Auslaßstutzen aufweist, abtrennt.

Die trennbare Verschweißung im Kammertrennbereich kann dadurch getrennt werden, daß auf mindestens eine der angrenzenden Kammern Druck ausgeübt wird, beispielsweise mit der flachen Hand. Es ist jedoch auch möglich, die äußere Wandung im Kammertrennbereich mit einer Aufreißlasche, vorzugsweise mit zwei Aufreißlaschen, zu versehen.

Das Verfahren zur Herstellung des erfindungsgemäßen Mehrkammerbeutels ist dadurch gekennzeichnet, daß die Verschweißung im Außenrandbereich bei einer höheren Temperatur durchgeführt wird als im Kammertrennbereich.

Dazu ist es notwendig, daß das den Kammern zugewandte polymere Material, vorzugsweise die Innenschicht der Mehrschichtfolie, in ihrem molekularen Aufbau Kettenbereiche aufweist, die zur Schwingungsanregung eine hohe und in anderen Bereichen eine deutlich niedrigere (Wärme-) Energiezufuhr benötigen.

Dieser Aufbau führt dazu, daß beim Verschweißen bei der niedrigen Temperatur lediglich die Bereiche aufschmelzen, die zum Aufschmelzen eine niedrige Energiezufuhr benötigen. Man spricht von einem partiellen Aufschmelzen. Bei der höheren Temperatur schmelzen dagegen sowohl die Bereiche, die eine niedrige Energiezufuhr benötigen, als auch die Bereiche, die eine hohe Energiezufuhr benötigen. Es kommt zu einem mehr oder weniger vollständigen Aufschmelzen der Innenschicht. Durch das Verschweißen im Bereich der niedrigen Temperatur ergibt sich eine trennbare, und im Bereich der höheren Temperatur eine nicht trennbare Verbindung.

Zur Herstellung einer Folien-Innenschicht, mit den oben beschriebenen Eigenschaften, sind prinzipiell folgende Möglichkeiten gegeben:
1. Polymerketten hoher und niedriger Anregungs- bzw. Schmelztemperatur sind in einem Polymer integriert.
2. Sowohl das Matrix-Polymer als auch das Phasen-Polymer weisen Kettenbereiche mit hoher und niedriger Anregungs- bzw. Schmelztemperatur auf.
3. Polymere mit hoher Anregungs- bzw. Schmelztemperatur bilden eine Matrix, in die ein weiteres Polymer eingebettet ist, welches Kettenbereiche mit niedriger Anregungs- bzw. Schmelztemperatur aufweist. Dieses sogenannte Matrix-Phasen-Polymer-System ist bevorzugt.

Die Verschweißung bei der höheren Temperatur wird im Außenrandbereich angewendet, da sich daraus nicht mehr trennbare Verbindungen ergeben. Die Verschweißung im Außenrandbeneich wird dabei vorzugsweise bei einer Temperatur im Bereich oberhalb von 128° bis 150°C durchgeführt.

Die Verschweißung bei der niedrigen Temperatur wird im Kammertrennbereich angewendet, da sich dadurch trennbare Verbindungen ergeben. Die Verschweißung im Kammertrennbereich wird dabei vorzugsweise bei einer Temperatur von ≤ 128°C durchgeführt.

Die untere Temperaturgrenze, bei der die Verschweißung im Kammertrennbereich durchgeführt werden kann, um wieder trennbare Schweißnähte herzustellen, variiert in Abhängigkeit vom verwendeten Folienmaterial der Innenschicht.

Die Dauer des Verschweißvorganges liegt vorzugsweise im Bereich von 1 bis 8 Sekunden und die während des Schweißvorganges auf die zu verschweißenden Bereiche ausgeübte Flächenpressung liegt vorzugsweise im Bereich von 0,1 bis 3 N/mm². Für die beiden genannten Parameter sind jedoch auch Werte außerhalb der genannten Bereiche möglich. Die beiden Parameter sind somit nicht auf die bevorzugten Bereiche beschränkt.

Der oben beschriebene Mehrkammerbeutel aus der erfindungsgemäßen Folie kann zur Herstellung von Mischlösungen, beispielsweise für die Dialyse, Infusion oder Ernährung verwendet werden, wobei unter dem Begriff Mischlösungen nicht nur eine Mischung aus Lösungen, also Flüssigkeiten, zu verstehen ist, sondern auch, das Mischen einer Lösung mit einem Feststoff.

Weitere Einzelheiten, Merkmale und Vorteile der Erfindung ergeben sich aus der nachfolgenden Beschreibung von Ausführungsbeispielen anhand der Zeichnungen.

Es zeigt
- Figur 1: eine schematisch vereinfachte Darstellung eines nicht erfindungsgemäßen Mehrkammerbeutels aus der erfindungsgemä- β en Folie;
- Figur 2: eine schematisch vereinfachte Darstellung einer weiteren bevor- zugten Ausführungsform eines Mehrkammerbeutels aus der erfin- dungsgemäßen Folie;
- Figur 3: graphisch die Ergebnisse des Zugversuches einer Folie gemäß Beispiel 1;
- Figur 4a: den Aufbau einer versiegelten Mehrschichtfolie im Außenrandbe- reich, die nachträglich nicht trennbar ist;
- Figur 4b: den Aufbau einer versiegelten Mehrschichtfolie im Kammertrenn- bereich, die nachträglich trennbar ist.

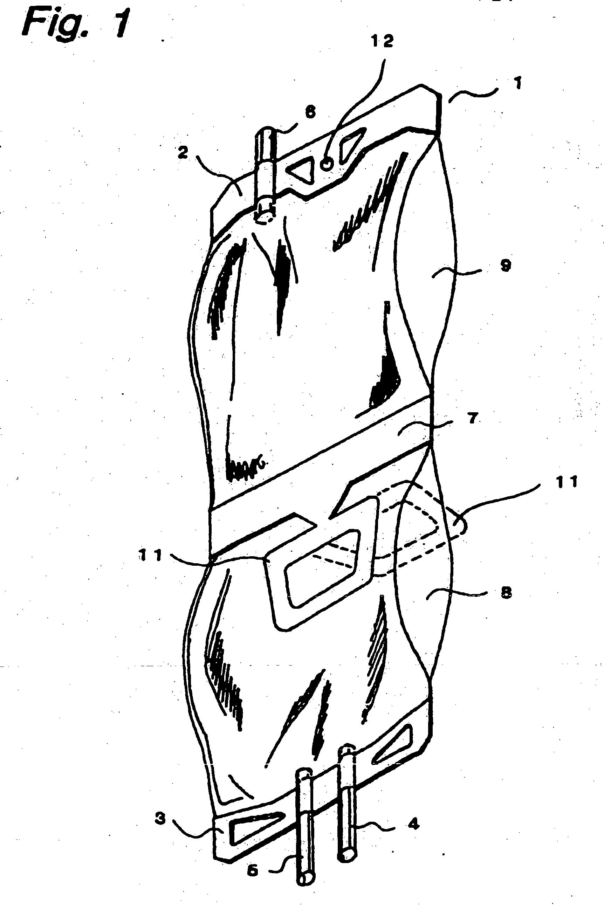

In Figur 1 ist ein Mehrkammerbeutel 1, gemäß einer nicht erfindungsgemäßen Ausfuhrungsform welche als Zweikammerbeutel mit zwei Beutelkammern 8 und 9 ausgebildet ist.

Der Beutel 1 wird aus einer gefalteten Blasfolie gebildet. Die beiden Kammern 8 und 9 werden gebildet durch Schweißnähte 2 und 3 (sogenannte Schweißbalken) in dem Außenrandbereich und durch die Schweißnaht 7 im Kammertrennbereich.

In beiden Schweißnähten im Außenrandbereich ist jeweils ein Stutzen 5, 6 eingeschweißt, die zum Befüllen der Beutelkammern dienen können. Zusätzlich weist die Schweißnaht 3 im Außenrandbereich der Kammer 8 einen weiteren Stutzen 4 auf, der als Auslaß gedacht ist.

Die Schweißnaht 7 weist an ihrer Außenseite zur leichteren Handhabung zwei Aufreißlaschen 10 auf.

Ferner ist die Schweißnaht 2, die der Schweißnaht 3, die den Auslaufstutzen aufweist, gegenüberliegt, mit einer Aufhängevorrichtung 11 in Form einer Öffnung ausgestattet.

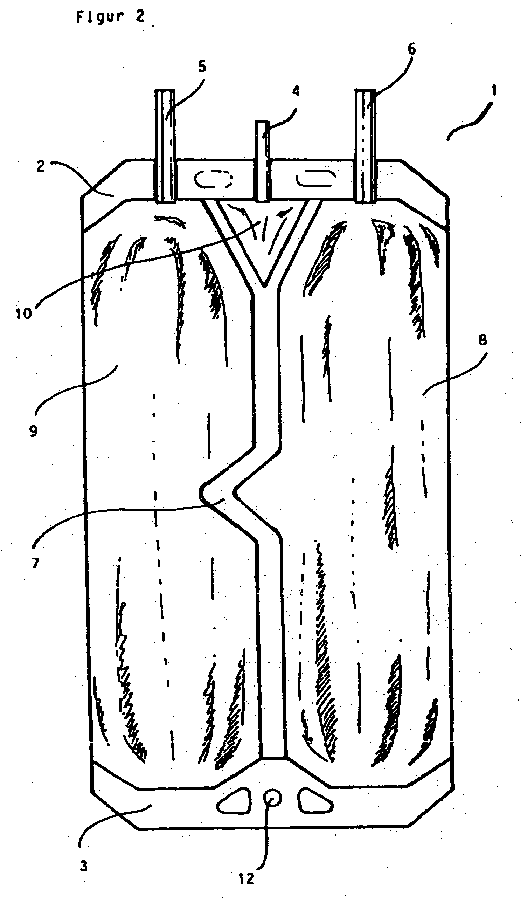

Figur 2 zeigt einen erfindungsgemäßen Dreikammerbeutel,

Der Beutel 1 wird ebenfalls aus einer gefalteten Blasfolie gebildet. Die Kammern 8, 9 und 10 werden durch Schweißnähte 2 und 3 im Außenrandbereich, sowie durch eine Schweißnaht 7 im Kammertrennbereich gebildet. Die Schweißnaht 7 im Kaumertrennbereich ist dabei so ausgebildet, daß sie zum einen die beiden befüllbaren Kammern längs der Blasfolie trennt und zum anderen eine leere Kammer 10 ausbildet.

Die beiden Kammern 8 und 9 können durch Füllstutzen 5 und 6, die in der Schweißnaht 2 des Außenrandbereichs eingeschweißt sind, befüllt werden. In der gleichen Schweißnaht 2 des Außenrandbereichs ist ebenfalls der Auslaßstutzen 4 eingeschweißt, der mit der leeren Kammer 10, die von den befüllbaren Kammern 8 und 9 durch die Schweißnaht 7 getrennt ist, verbunden ist.

Ferner ist in der Schweißnaht 3, die der Schweißnaht 2, die den Auslaufstutzen aufweist, gegenüberliegt, eine Aufhängevorrichtung 12 in Form einer Öffnung vorgesehen.

Der Aufbau der Folie wird in den folgenden drei Beispielen anhand von 3-Schicht-Folien, die eine bevorzugte Ausführungsform darstellen, näher beschrieben. Dabei wird in den Beispielen die Außenschicht der Folie, das ist die den Kammerbeuteln abgewandte Seite, als Schicht 1 bezeichnet. Schicht 2 ist die Mittelschicht und Schicht 3 ist die den Kammern zugewandte Seite der Folien.

### Beispiel 1: 3-Schicht-Folie ohne Gasbarriere

Die Folie wird auf einer 3-Schicht Coextrusions-Blasfolienanlage hergestellt. Die Extruder haben 30D Länge und sind mit Misch- und Scherteil ausgerüstet. Das Coextrusions-Werkzeug ist als Wendelverteiler-Werkzeug ausgelegt.

| Folienaufbau: | | | | |
|---|---|---|---|---|
| | Kunststofftyp Bezeichnung | | Hersteller | MFI |
| Schicht 1 | 100 % PP-Homopolymer | Novolen 1302H | BASF | 1,8 (230/2.16) |
| Schicht 2 | 70 % VLDPE | Tearnex 1000F | DSM | 3.0 (190/2.16) |
| | 30 % PP-Homopolymer | Novolen 1302H | BASF | 1.8 (230/2.16) |
| Schicht 3 | 70 % PP-Random-Copo. | Novolen 3200HX | BASF | 1.8 (230/2.16) |
| | 30 % SEBS-Compound | 90% Kraton G1650 | Shell | |
| | | 10% Ondina G100 | Shell | |

Das SEBS Compound wurde zuvor in einem separaten Verarbeitungsschritt compoundiert und granuliert.

Ondina G100 ist ein medizinisches Weißöl auf paraffinischer Basis.

**Beispiel 2:** wie Beispiel 1, außer, daß in Schicht 3 als SEBS-Compound ein commerziell erhältliches Produkt der Firma GW-Kraiburg eingesetzt wurde.

| | Kunststofftyp | Bezeichnung | Hersteller | MFI |
|---|---|---|---|---|
| Schicht 3 | 70 % PP-R | Novolen 3200HX | BASF | 1.8 (230/2.16) |
| | 30 % SEBS-Compound | TF6AAF | GW-Kraiburg - | |

Die Folien werden dabei durch Coextrusion hergestellt. Je nach Folienaufbau werden Ausgangsrohstoffe und Blends in verschiedenen Schichten kombiniert. Ziel hierbei ist es neben der eigentlichen Erfindung "Trennaht" Eigenschaften wie Schlagzähigkeit z.B. Fall aus 2 m Höhe und Gasbarriere zu erhalten.

Eine 3-Schicht-Folie, die entsprechend Beispiel 1 und 2 aufgebaut ist, wurde einem Schweißversuch unterworfen, der im folgenden näher beschrieben wird. Als Schweißvorrichtung wurden zwei Schweißbalken mit einer Länge von 220 mm und einer Breite von 10 bis 15 mm verwendet. Die Länge der Schweißbalken (220 mm) muß dabei mindestens genauso lang, vorzugsweise etwas länger sein als die herzustellende Schweißnaht (180 mm). Pro Schweißbalken wurden vier Heizelemente mit einer Leistung von maximal je 200 Watt in gleichen Abständen und 25 mm unter der Siegelfläche angebracht. Die Regelung erfolgte über einen Temperatursensor des Typs PT 100 mittig zwischen zwei Heizelementen. Die zu verschweißende Folie wurde zwischen die beiden Schweißbalken gebracht, von denen der eine fest und der andere beweglich angeordnet ist. Der bewegliche Schweißbalken wurde mittels eines Kolbens (Durchmesser 100mm) unter einem bestimmten Druck gegen den fest angeordneten Schweißbalken gedrückt.

Die Schweißzeit betrug einheitlich 6 Sekunden. Die Temperatur wurde von 115°C bis 131°C, in Schritten von 2°C, variiert. Der Druck konnte von 1 bar bis 6 bar, in Schritten von 1 bar, variiert werden. Aus den verschiedenen Drücken ergeben sich aufgrund der Geometrie der Schweißvorrichtung (z.B. Länge und Breite der Schweißbalken sowie Kolbendurchmesser) und der Folie (z.B. Länge und Breite der Schweißnaht) Werte für die Flächenpressung der zu verschweißenden Bereiche, die im Bereich von 0,1 bis 3 N/mm² liegen.

Die Anfangstemperatur der Versuchsreihe wurde auf 115°C festgelegt, da die Folie im darunterliegenden Temperaturbereich keine Haftung aufwies. Bei einer Verschweißungstemperatur (Siegeltemperatur) von 131°C und höher ist die Haftung der Folienflächen wiederum so groß, daß bei den Zugversuchen die Schweißnaht unlösbar war und die Folie zerriß.

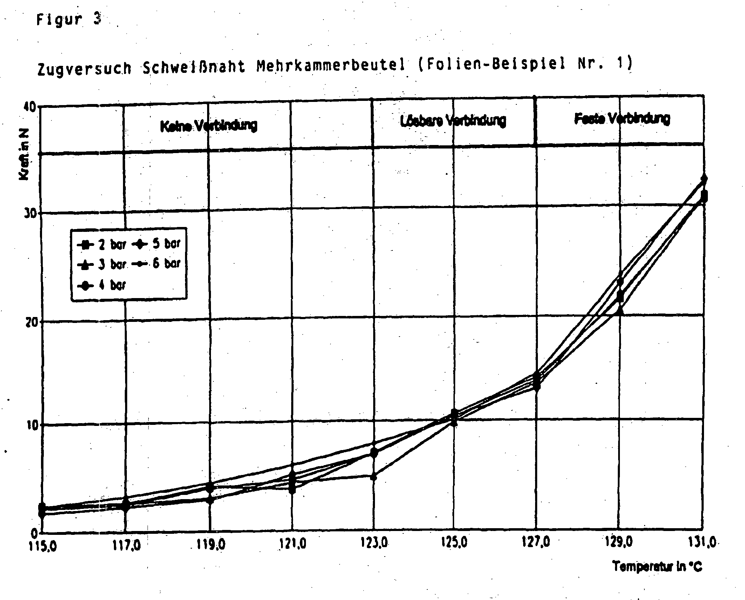

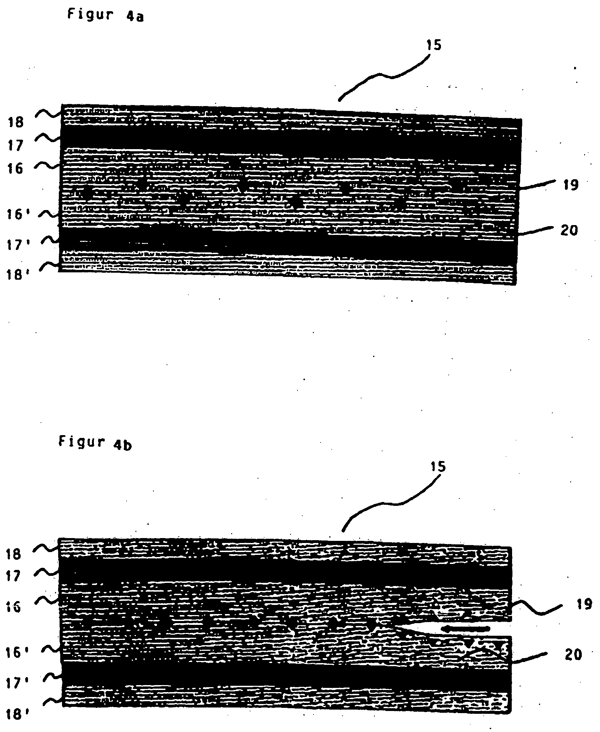

Die bei verschiedenen Drücken und im Temperaturbereich von 115°C bis 131°C verschweißten Nähte der Mehrkammerbeutel wurden einem Zugversuch gemäß DIN 53457 unterworfen. Die Ergebnisse dieser Zugversuche sind in den Figuren 3 und 4 graphisch dargestellt.

Wie der Figur 3 zu entnehmen ist, ergibt sich für die untersuchte Folie, die gemäß Beispiel 1 hergestellt wurde, folgendes Bild:
- Im Temperaturbereich von 115°C bis 122°C ist die Schweißnaht mit einer Kraft, die kleiner als 5 N ist, trennbar, d.h. die Verbindung der Siegelfläche ist zu gering, um als Schweißnaht in erfindungsgemäßen Mehrkammerbeuteln verwendet werden zu können.
- Im Temperaturbereich oberhalb von 128°C bis 131°C ist die Schweißnaht nur mit einer Kraft von über 20 N trennbar, d.h. die Siegelflächen sind fest miteinander verbunden.
- Im Temperaturbereich von 123°C bis 128°C ist die Schweißnaht mit einer Kraft im Bereich von 5 bis 20 N trennbar. In diesem Temperaturbereich liegt die optimale Peel-Fähigkeit, d.h. eine Verbindung, die nachträglich wieder lösbar ist.

Die Druckangaben sind die bei der Verschweißung angewendeten Drücke. Die Eigenschaften der verschweißten Naht sind jedoch im wesentlichen unabhängig von dem bei der Verschweißung angewendeten Druck, wie in Figur 3 leicht zu erkennen ist.

Für die untersuchte Folie ergeben sich folgende Temperaturbereiche:
- Eine Schweißnaht, die unterhalb von 115°C verschweißt wurde, ist mit einer Kraft, die kleiner als 5 N ist, trennbar, d.h. die Verbindung der Siegelfläche ist zu gering, um als Schweißnaht in einem erfindungsgemäßen Mehrkammerbeutel verwendet werden zu können.
- Eine Schweißnaht, die im Temperaturbereich von 115 bis 128°C verschweißt wurde, ist mit einer Kraft im Bereich von 5 bis 20 N trennbar, d.h. eine solche Schweißnaht wird erfindungsgemäß im Kammertrennbereich eingesetzt, da diese Verbindung nachträglich wieder lösbar ist.
- Eine Schweißnaht, die im Temperaturbereich oberhalb von 128°C verschweißt wurde, ist nur mit einer Kraft von über 20 N trennbar, d.h. eine solche Schweißnaht wird erfindungsgemäß im Außenrandbereich eingesetzt, da diese Siegelflächen fest miteinander verbunden sind.

Figur 4a zeigt eine gemäß Beispiel 1 aufgebaute 3-Sehicht-Folie mit Innenschichten 16 und 16', mit Mittelschichten 17 und 17' sowie Außenschichten 18 und 18', die bei einer Temperatur von über 128°C verschweißt wurde. Die beiden Innenschichten 16 und 16' sind, wie der Graphik zu entnehmen ist, so miteinander verschmolzen, daß eine nichttrennbare Verbindung entstanden ist.

Figur 4b zeigt eine gemäß Beispiel 1 aufgebaute 3-Schicht-Folie, wie sie in Figur 4a beschrieben wurde. Der Unterschied zu Figur 4a besteht lediglich darin, daß die Folie im Temperaturbereich von 123°C bis 128°C verschweißt wurde. In diesem Fall sind die beiden Innenschichten 16 und 16', wie der Graphik zu entnehmen ist, so miteinander verschmolzen, das eine nachträgliche trennbare Verbindung entstanden ist.

## Patentansprüche

1. Medizinischer Mehrkammerbeutel (1) aus einem polymeren Material zur Herstellung von medizinischen Mischlösungen mit im Außenbereich verschweißten Rändern (2, 3), drei Kammern (8, 9, 10), von denen zumindest zwei Kammern (8, 9) zur Aufnahme einer Mischlösungskomponente vorgesehen sind und die durch eine aufreißbare Schweißnaht (7) voneinander getrennt sind, und einem Auslassstutzen (4), der in dem Außenrand (2) eingeschweißt ist,
**dadurch gekennzeichnet, dass**
die Schweißnaht (7) zusätzlich so ausgebildet ist, dass eine dritte Kammer (10) entsteht, mit der der Auslassstutzen (4) in Verbindung steht,
wobei die Schweißnaht (7) weiterhin so ausgebildet ist, dass bei ihrem Aufreißen zuerst die die Mischlösungskomponenten enthaltenden Kammern (8, 9) und anschließend die dritte Kammer (10) geöffnet werden, wobei das polymere Material eine Mehrschichtfolie ist, wobei
die den Kammern zugewandte Schicht der Mehrschichtfolie im wesentlichen zwei Komponenten aufweist, nämlich ein Matrix-Polymer und ein Phasen-Polymer, und wobei die Folie so ausgebildet ist, dass sich beim Verschweißen bei einer ersten Temperatur eine peelfähige Naht und beim Verschweißen bei einer zweiten Temperatur eine feste Naht ergibt..

2. Medizinischer Mehrkammerbeutel gemäß Anspruch 1, **dadurch gekennzeichnet, dass** die Mehrschichtfolie eine coextrudierte Mehrschichtfolie ist.

3. Medizinischer Mehrkammerbeutel gemäß Anspruch 1 oder 2, **dadurch gekennzeichnet, dass** die Mehrschichtfolie zwei bis sieben Schichten aufweist.

4. Medizinischer Mehrkammerbeutel gemäß einem der vorherigen Ansprüche, **dadurch gekennzeichnet, dass** er unter Verwendung von einzelnen Folienblättern hergestellt ist.

5. Medizinischer Mehrkammerbeutel gemäß einem der Ansprüche 1 bis 4, **dadurch gekennzeichnet, dass** er unter Verwendung einer Blas- oder Schlauchfolie hergestellt ist.

6. Medizinischer Mehrkammerbeutel gemäß einem der vorherigen Ansprüche, **dadurch gekennzeichnet, dass** die äußere Wandung im Kammertrennbereich mit mindestens einer Aufreißlasche (10), vorzugsweise mit zwei Aufreißlaschen (10), versehen ist.

7. Medizinischer Mehrkammerbeutel gemäß einem der Ansprüche 1 bis 6, **dadurch gekennzeichnet, dass** er durchsichtig ist.

8. Medizinischer Miehrkammerbeutel gemäß einem der Ansprüche 1 bis 6, **dadurch gekennzeichnet, dass** er hitzesterilisierbar ist.

9. Verwendung eines medizinischen Mehrkammerbeutels gemäß einem der vorherigen Ansprüche zur Herstellung von Mischlösungen für die Dialyse, Infusion oder Ernährung.

## Claims

1. Medical multi-chamber bag (1) of a polymeric material for the preparation of medical mixed solutions, with edges (2, 3), which are welded in the outer area, at least three chambers (8, 9, 10) of which at least two chambers (8, 9) are provided to accommodate a component of a mixed solution, and which are separated by a rupturable weld seam (7), and an outlet (4), which is welded into the outer edge (2),
**characterized in that**
the weld seam (7) is additionally configured such that a third chamber (10) is formed, with which the outlet (4) is in connection,
wherein the weld seam (7) is further configured such that when ruptured, first the chambers (8, 9), containing the components of the mixed solution and then the third chamber (10), will be opened,
wherein the polymeric material is a multilayer film,
wherein the layer of the multilayer film, which faces the chambers, has essentially two components, namely a matrix-polymer and a phase-polymer, and
wherein the film is configured such that when welded at a first temperature a peelable weld seam is formed, and when welded at a second temperature a solid weld seam is formed.

2. Medical multi-chamber bag according to claim 1, **characterized in that** the multilayer film is a coextruded multilayer film.

3. Medical multi-chamber bag according to claim 1 or 2, **characterized in that** the multilayer film has two to seven layers.

4. Medical multi-chamber bag according to any one of the preceding claims, **characterized in that** the bag is made, using individual film sheets.

5. Medical multi-chamber bag according to any one of the claims 1 to 4, **characterized in that** the bag is made, using a blown or tubular film.

6. Medical multi-chamber bag according to any one of the preceding claims, **characterized in that** the outer wall is provided in the chamber separation area with at least one tear flap (10), preferably with two tear flaps (10).

7. Medical multi-chamber bag according to any one of the claims 1 to 6, **characterized in that** the bag is transparent.

8. Medical multi-chamber bag according to any one of the claims 1 to 6, **characterized in that** the bag can be used for heat-sterilization.

9. Use of a medical multi-chamber bag according to any one of the preceding claims for the preparation of mixed solutions for dialysis, infusion, or nutrition.

## Revendications

1. Sac médical (1) à chambres multiples constitué d'une matière polymère pour la préparation de solutions mixtes médicales, présentant des bordures (2, 3) soudées dans la zone extérieure, au moins trois chambres (8, 9, 10), parmi lesquelles au moins deux chambres (8, 9) sont prévues pour la réception d'une composante de solution mixte, et qui sont séparées les unes des autres par un cordon de soudure déchirable (7), et une tubulure de sortie (4) qui est soudée dans la bordure extérieure (2),
**caractérisé en ce que**
le cordon de soudure (7) est additionnellement ainsi réalisé qu'il en résulte une troisième chambre (10) avec laquelle la tubulure de sortie (4) est en communication,
dans lequel le cordon de soudure (7) est en outre ainsi réalisé que lorsqu'il est déchiré, les chambres qui contiennent les composantes de solution mixte (8, 9) sont tout d'abord ouvertes, et ensuite la troisième chambre (10) est ouverte ;
dans lequel la matière polymère est une feuille multicouche,
dans lequel la couche de la feuille multicouche tournée vers les chambres comprend sensiblement deux composantes, à savoir un polymère de matrice et un polymère de phase, et
dans lequel la feuille est ainsi réalisée que lors d'une soudure à une première température il en résulte un cordon capable d'être pelé, et lors d'une soudure à une seconde température il en résulte un cordon ferme.

2. Sac médical à chambres multiples selon la revendication 1, **caractérisé en ce que** la feuille multicouche est une feuille multicouche coextrudée.

3. Sac médical à chambres multiples selon la revendication 1 ou 2, **caractérisé en ce que** la feuille multicouche comprend de deux à sept couches.

4. Sac médical à chambres multiples selon l'une des revendications précédentes, **caractérisé en ce qu'**il est réalisé en utilisant des panneaux de feuilles individuels.

5. Sac médical à chambres multiples selon l'une des revendications 1 à 4, **caractérisé en ce qu'**il est réalisé en utilisant une feuille gonflable ou une feuille en forme de tuyau.

6. Sac médical à chambres multiples selon l'une des revendications précédentes, **caractérisé en ce que** la paroi extérieure est pourvue, dans la zone de séparation des chambres, d'au moins une patte d'arrachement (10), de préférence de deux pattes d'arrachement (10).

7. Sac médical à chambres multiples selon l'une des revendications 1 à 6, **caractérisé en ce qu'**il est transparent.

8. Sac médical à chambres multiples selon l'une des revendications 1 à 6, **caractérisé en ce qu'**il peut être stérilisé à la chaleur.

9. Utilisation d'un sac médical à chambres multiples selon l'une des revendications précédentes pour la préparation de solutions mixtes pour dialyse, perfusion ou alimentation.
